(19) Europäisches Patentamt
European Patent Office
Office européen des brevets

(11) EP 0 874 615 B1

(12) # EUROPÄISCHE PATENTSCHRIFT

(45) Veröffentlichungstag und Bekanntmachung des Hinweises auf die Patenterteilung:
**02.04.2003 Patentblatt 2003/14**

(21) Anmeldenummer: **97910383.5**

(22) Anmeldetag: **29.09.1997**

(51) Int Cl.$^7$: **A61K 7/13**

(86) Internationale Anmeldenummer:
**PCT/EP97/05338**

(87) Internationale Veröffentlichungsnummer:
**WO 98/019661 (14.05.1998 Gazette 1998/19)**

(54) **FÄRBEMITTEL ZUR FÄRBUNG VON KERATINISCHEN FASERN**

COLORANTS FOR COLOURING KERATIN FIBRES

COLORANT S'UTILISANT DANS LA COLORATION DE FIBRES KERATINIQUES

(84) Benannte Vertragsstaaten:
**DE ES FR GB IT**

(30) Priorität: **05.11.1996 DE 19645541**

(43) Veröffentlichungstag der Anmeldung:
**04.11.1998 Patentblatt 1998/45**

(73) Patentinhaber: **Wella Aktiengesellschaft**
**64274 Darmstadt (DE)**

(72) Erfinder: **BRAUN, Hans-Jürgen, Dr.**
**3182 Überstorf (CH)**

(56) Entgegenhaltungen:
**DE-A- 3 834 142** **DE-A- 3 942 294**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 874 615 B1

Printed by Jouve, 75001 PARIS (FR)

## Beschreibung

Die vorliegende Erfindung beschreibt eine Zusammensetzung zur Färbung von keratinischen Fasern, insbesonders von menschlichen Haaren mit einem Gehalt an einem p-Aminophenol, einem m-Aminophenol, einem m-Phenylendiamin und einem 2-Amino-6-chlor-4-nitrophenol-derivat.

Auf dem Gebiet der Haarfärbung haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines Oxidationsmittels.

An Oxidationsfarbstoffe, die zur Färbung menschlicher Haare verwendet werden sollen, wird eine Vielzahl von Anforderungen gestellt. Die Farbstoffe müssen in toxikologischer und dermatologischer Hinsicht unbedenklich sowie nicht sensibilisierend sein und Färbungen in der gewünschten Intensität ermöglichen.

Femer wird für die erzielten Haarfärbungen eine gute Licht-, Dauerwell-, Säure- und Reibeechtheit gefordert. Die Haarfärbungen sollen auch ohne Einwirkung von Licht, Reibung und chemischen Mitteln über einen Zeitraum von mindestens vier bis sechs Wochen stabil bleiben.Außerdem ist es erforderlich, daß durch Kombination geeigneter Entwickler- und Kupplerkomponenten eine breite Palette verschiedener Farbnuancen erzeugt werden kann.

Ein weiteres Problem besteht in der Praxis bezüglich des unterschiedlichen Aufziehverhaltens der Farbstoffe in Abhängigkeit von der Haarbeschaffenheit, wodurch eine ungleichmäßige Anfärbung der Haare erfolgt. Normalerweise neigen die verwendeten Farbstoffe dazu, auf geschädigte Haarpartien stärker aufzuziehen als auf ungeschädigte Haarpartien. Aus diesem Grunde werden in der Regel die durch übliche Alterungsprozesse und Umwelteinflüsse (zum Beispiel Sonnenlicht, Haarwäsche, Färbe- und Dauerwellbehandlungen) stärker geschädigten Haarspitzen intensiver gefärbt als die weniger geschädigten Haarlängen und der Haaransatz, wodurch ein unnatürliches, ungleichmäßiges und völlig unbefriedigendes Färbeergebnis erhalten wird. Dieses Verhalten macht sich insbesondere bei hellen Nuancen sehr störend bemerkbar.

Es bestand daher die Aufgabe, ein Oxidationshaarfärbemittel zur Verfügung zu stellen, das eine gleichmäßige Färbung der Haare von den Haarspitzen bis zum Haaransatz im Naturtonbereich ermöglicht, und gleichzeitig wenig oder gar nicht mutagen und sensibilisierend sowie physiologisch gut verträglich ist.

In der EP-OS 0 634 162 wird die Kombination aus einem p-Aminophenol, einem m-Aminophenol und einem m-Phenylendiamin zur Färbung von Haaren in modischen, leicht rötlichen Farbtönen empfohlen. Diese Kombination ergibt jedoch auf ungleichmäßig geschädigten Haaren ein unbefriedigendes Farbresultat. Außerdem sind helle Nuancen damit sehr schwierig nuancierbar.

Aus der DE-OS 39 42 294 sind zudem Oxidationsfärbemittel bekannt, welche 2-Amino-6-chlor-4-nitrophenol enthalten.

Es wurde nun überraschenderweise gefunden, daß die geschilderten Nachteile vermieden werden können, wenn für die oxidative Haarfärbung eine Farbträgermasse enthaltend eine Kombination aus

- mindestens einem p-Aminophenolderivat, welches ausgewählt ist aus 4-Amino-2-methyl-phenol, 4-Amino-3-methyl-phenol, 4-Amino-2-hydroxymethyl-phenol, 4-Amino-2-methoxy-methyl-phenol und 4-Amino-2-aminomethylphenol,
- mindestens einem 5-Amino-2-methyl-phenol gemäß der allgemeinen Formel (I),

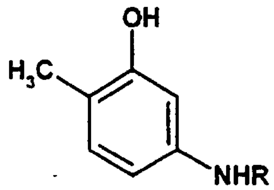

(I)

,

worin R Wasserstoff, eine geradkettige oder verzweigte Alkylgruppe mit 1 bis 4 Kohlenstoffatomen, eine geradkettige oder verzweigte Hydroxyalkylgruppe mit 2 bis 4 Kohlenstoffatomen oder eine geradkettige oder verzweigte Polyhydroxyalkylgruppe mit 3 bis 4 Kohlenstoffatomen bedeutet,

- mindestens einem m-Phenylendiaminderivat gemäß der allgemeinen Formel (II),

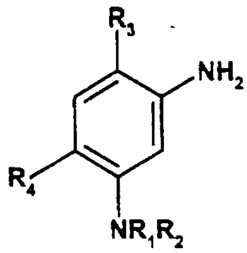

(II)

,

worin $R_1$ und $R_2$ unabhängig voneinander Wasserstoff, eine geradkettige oder verzweigte Alkylgruppe mit 1 bis 4 Kohlenstoffatomen, eine geradkettige oder verzweigte Hydroxyalkylgruppe mit 2 bis 4 Kohlenstoffatomen oder eine geradkettige oder verzweigte Polyhydroxyalkylgruppe mit 3 bis 4 Kohlenstoffatomen bedeuten,
$R_3$ Wasserstoff, eine geradkettige oder verzweigte Alkylgruppe mit 1 bis 6 Kohlenstoffatomen, eine geradkettige oder verzweigte Alkoxygruppe mit 1 bis 4 Kohlenstoffatomen, eine geradkettige oder verzweigte Hydroxyalkylgruppe mit 2 bis 4 Kohlenstoffatomen oder eine geradkettige oder verzweigte Polyhydroxyalkylgruppe mit 3 bis 4 Kohlenstoffatomen bedeutet, und
$R_4$ unabhängig von $R_1$, $R_2$ und $R_3$ Wasserstoff, eine geradkettige oder verzweigte Alkylgruppe mit 1 bis 6 Kohtenstoffatomen, eine geradkettige oder verzweigte Alkoxygruppe mit 1 bis 4 Kohlenstoffatomen, eine geradkettige oder verzweigte Hydroxyalkylgruppe mit 2 bis 4 Kohlen stoffatomen oder eine geradkettige oder verzweigte Polyhydroxyalkyl gruppe mit 3 bis 4 Kohlenstoffatomen bedeutet,
und

- mindestens einem 2-Amino-6-Chlor-4-nitrophenol gemäß der allgemeinen Formel (III),

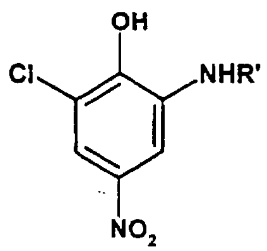

(III)

,

worin R' gleich Wasserstoff, einer geradkettigen oder verzweigten Alkyl gruppe mit 1 bis 6 Kohlenstoffatomen, einer geradkettigen oder verzweigten Hydroxyalkylgruppe mit 2 bis 4 Kohlenstoffatomen oder einer geradkettigen oder verzweigten Polyhydroxyalkylgruppe mit 3 bis 4 Kohlenstoffatomen ist, verwendet wird.

Bevorzugte Verbindungen der allgemeinen Formel (I) sind 5-Amino-2-methyl-phenol, 5-Methylamino-2-methyl-phenol und 5-[(2'-Hydroxyethyl)amino]-2-methyl-phenol.

Bevorzugte Verbindungen der allgemeinen Formel (II) sind 2,4-Diamino-1-(2',3'-dihydroxypropoxy)benzol, 2-(2',4'-Diaminophenoxy)ethanol, 1,3-Bis(2',4'-diaminophenoxy)propan, 2-Amino-4-[(2'-hydroxyethyl)amino]anisol, 1,5-Bis(2'-hydroxyethoxy)-2,4-diaminobenzol und m-Phenylendiamin.

Bevorzugte Verbindungen der allgemeinen Formel (III) sind 2-Amino-6-chlor-4-nitrophenol, 2-Chlor-6-ethylamino-4-nitrophenol und 2-Chlor-6-((2'-hydroxyethyl)amino)-4-nitrophenol.

Die erfindungsgemäße Farbstoffkombination ermöglicht eine vom Haaransatz bis zur Haarspitze gleichmäßige, modische Färbung mit rötlichen Reflexen, insbesondere auch bei helleren Nuancen. Die vorzüglichen Eigenschaften der neuen Farbstoffkombination zeigen sich besonders auf durch Licht und Wetter geschädigtem oder auf dauergewellten Haaren.

Zur Abrundung des Farbergebnisses sowie zur Erzeugung von speziellen Farbeffekten können dem Haarfärbemittel weitere Oxidationsfarbvorstufen wie Derivate des p-Phenylendiamins, Resorcins und 1,3-Benzodioxols, sowie direktziehende Farbstoffe, wie zum Beispiel Azofarbstoffe, Anthrachinone oder Nitrobenzolderivate, zugesetzt werden.

Die vorstehend beschriebene Farbträgermasse wird zur Färbung gemeinsam mit einem Oxidationsmittel in

einer geeigneten kosmetischen Zubereitung appliziert.

Der Gegenstand der vorliegenden Anmeldung betrifft daher auch ein Mittel zur oxidativen Färbung von Haaren, welches unmittelbar vor der Anwendung durch Vermischen der erfindungsgemäßen Farbträgermasse mit einem Oxidationsmittel hergestellt wird.

Die erfindungsgemäße Farbträgermasse enthält die vorstehend beschriebenen erfindungsgemäßen Farbvorstufen als solche oder in Form von physiologisch verträglichen Salzen, beispielsweise Hydrochloriden, Sulfaten oder Tartraten oder im Fall von Phenolen als Alkaliphenolate.

Die Gesamtkonzentration an Farbvorstufen beträgt etwa 0,1 bis 10 Gewichtsprozent, vorzugsweise etwa 0,2 bis 6 Gewichtsprozent.

Zusätzlich können in der Farbträgermasse übliche kosmetische Zusätze, beispielsweise Antioxidantien wie Ascorbinsäure, Thioglykolsäure oder Natriumsulfit; Parfümöle; Komplexbildner; Netzmittel und Emulgatoren; Verdikker; Pflegestoffe und andere, enthalten sein.

Die Zubereitungsform für die Farbträgermasse sowie für das gebrauchsfertige Oxidationshaarfärbemittel kann beispielsweise eine Lösung, insbesondere eine wäßrige oder wäßrig-alkoholische Lösung sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.

Übliche Zusätze in Lösungen, Cremes, Emulsionen oder Gelen sind zum Beispiel Lösungsmittel wie Wasser, niedere aliphatische Alkohole, beispielsweise Ethanol, n-Propanol und Isopropanol oder Glykole wie Glycerin und 1,2-Propylenglykol, weiterhin Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohle, oxethylierte Nonylphenole, Fettsäurealkanolamide, oxethylierte Fettalkohole, oxethylierte Nonylphenole, Fettsäurealkanolamide, oxethylierte Fettsäureester, ferner Verdicker wie höhere Fettalkohole, Stärke oder Cellulosederivate, weiterhin Vaseline, Paraffinöl und Fettsäuren sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent (bezogen auf die Farbträgermasse), die Verdicker in einer Menge von etwa 0,1 bis 25 Gewichtsprozent (bezogen auf die Farbträgermasse) und die Pflegestoffe in einer Konzentration von etwa 0,1 bis 5.0 Gewichtsprozent (bezogen auf die Farbträgermasse).

Das gebrauchsfertige erfindungsgemäße Haarfärbemittel wird unmittelbar vor der Anwendung durch Vermischen der erfindungsgemäßen Farbträgermasse mit einem Oxidationsmittel hergestellt.

Die Farbträgermasse und das Oxidationsmittel werden hierbei im Gewichtsverhältnis von 5:1 bis 1:3 miteinander vermischt, wobei ein Gewichtsverhältnis von 1:1 bis 1:2 besonders bevorzugt ist.

Des pH-Wert des gebrauchsfertigen erfindungsgemäßen Haarfärbemittel stellt sich bei der Mischung der vorzugsweise alkalisch eingestellten Farbträgermasse mit dem meist sauer eingestellten Oxidationsmittel auf einen pH-Wert ein, der durch die Alkalimenge in der Farbträgermasse und die Säuremenge im Oxidationsmittel sowie durch das Mischungsverhältnis bestimmt wird. Der pH-Wert des gebrauchsfertigen Haarfärbemittels beträgt in der Regel 3 bis 11, vorzugsweise 5 bis 9.

Für die Einstellung des pH-Wertes der Farbträgermasse und des Oxidationsmittels können je nach dem gewünschten pH-Wert organische und anorganische Säuren, wie zum Beispiel Phosphorsäure, Ascorbinsäure oder Milchsäure, oder Alkalien wie zum Beispiel Monoethanolamin, Triethanolamin, 2-Amino-2-methyl-1-propanol, Ammoniak, Natronlauge, Kalilauge oder Tris(hydroxymethyl)-amino-methan, verwendet werden.

Für die Anwendung zur oxidativen Färbung von Haaren vermischt man die vorstehend beschriebene Farbträgermasse unmittelbar vor dem Gebrauch mit einem Oxidationsmittel und trägt eine für die Haarfärbebehandlung ausreichende Menge, je nach Haarfülle im allgemeinen etwa 60 bis 200 Gramm, des erhaltenen gebrauchsfertigen Oxidationshaarfärbemittels auf das Haar auf.

Als Oxidationsmittel kommen insbesondere Wasserstoffperoxid oder dessen Additionsverbindungen an Harnstoff, Melamin oder Natriumbromat in Form einer 1- bis 12prozentigen, vorzugsweise 6prozentigen, wäßrigen Lösung in Betracht, wobei Wasserstoffperoxid besonders bevorzugt wird.

Man läßt das erfindungsgemäße Haarfärbemittel bei 15 bis 50 Grad Celsius etwa 10 bis 45 Minuten lang, vorzugsweise 30 Minuten lang, auf das Haar einwirken, spült sodann das Haar mit Wasser und trocknet es. Gegebenenfalls wird im Anschluß an diese Spülung mit einem Shampoo gewaschen und/oder mit einer schwachen organischen Säure, wie zum Beispiel Zitronensäure oder Weinsäure, nachgespült. Anschließend wird das Haar getrocknet.

Alle Prozentangaben stellen soweit nicht anders angegeben Gewichtsprozente dar

Die nachfolgenden Beispiele sollen den Gegenstand näher erläutern, ohne ihn auf diese Beispiele zu beschränken.

**Beispiele**

**Beispiel 1:** Haarfärbelösung

| | |
|---|---|
| 1,2 g | 4-Amino-3-methyl-phenol |
| 1,3 g | 5-Amino-2-methyl-phenol |
| 0,5 g | 2-(2',4'-Diaminophenoxy)ethanol-dihydrochlorid |
| 0,2 g | 2-Amino-6-chlor-4-nitrophenol |
| 10,0 g | Isopropanol |
| 10,0 g | Laurylalkohol-diglykolether-sulfat-Natriumsalz (28prozentige wäßrige Lösung) |
| 10,0 g | Ammoniak (25prozentige wäßrige Lösung) |
| 0,3 g | Ascorbinsäure |
| 66,5 g | Wasser, vollentsalzt |
| 100,0 g | |

Vor der Anwendung werden 10 g Haarfärbelösung mit 10 g Wasserstoffperoxid-Lösung (6prozentige wäßrige Lösung) vermischt. Das erhaltene gebrauchsfertige Oxidationshaarfärbemittel wird auf durch Wind und Wetter geschädigte Haare aufgetragen. Nach einer Einwirkungszeit von 30 Minuten bei 40 °C werden die Haare mit Wasser gespült, schamponiert und getrocknet.

Das Haar hat einen gleichmäßigen kupferbraunen Farbton erhalten.

**Beispiel 2:** Haarfärbecreme

| | |
|---|---|
| 0,5 g | 4-Amino-3-methyl-phenol |
| 0,3 g | 2-(2',5'-Diaminophenyl)-ethanol-sulfat |
| 0,8 g | 5-Amino-2-methyl-phenol |
| 0,1 g | 1,3-Bis(2',4'-diaminophenoxy)propan-tetrahydrochlorid |
| 0,2 g | 2-Chlor-6-(ethylamino)-4-nitrophenol |
| 15,0 g | Cetylalkohol |
| 3,5 g | Laurylalkohol-diglykolether-sulfat-Natriumsalz (28prozentige wäßrige Lösung) |
| 3,0 g | Ammoniak (25prozentige wäßrige Lösung) |
| 0,3 g | Natrimsulfit, wasserfrei |
| 76,3 g | Wasser, vollentsalzt |
| 100,0 g | |

Vor der Anwendung werden 10 g Haarfärbecreme mit 10 g Wasserstoffperoxid-Lösung (6prozentige wäßrige Lösung) gemischt. Das erhaltene gebrauchsfertige Oxidationshaarfärbemittel wird auf durch Wind und Wetter geschädigte Haare aufgetragen. Nach einer Einwirkungszeit von 30 Minuten bei 40 °C werden die Haare mit Wasser gespült, schamponiert und getrocknet.

Das Haar ist gleichmäßig vom Ansatz bis zur Spitze in einem rotbraunen Ton gefärbt.

**Beispiel 3:** Haarfärbelösung

| | |
|---|---|
| 1,1 g | 4-Amino-3-methyl-phenol |
| 1,0 g | 5-Amino-2-methyl-phenol |
| 0,3 g | 1 ,5-Bis(2'-hydroxyethyl)-2,4-diaminobenzoldihydrochlorid |
| 0,2 g | 2-Amino-6-chlor-4-nitrophenol |
| 10,0 g | Isopropanol |

(fortgesetzt)

| | |
|---|---|
| 10,0 g | Laurylalkohol-diglykolether-sulfat-Natriumsalz (28prozentige wäßrige Lösung) |
| 10,0 g | Ammoniak (25prozentige wäßrige Lösung) |
| 0,3 g | Ascorbinsäure |
| 67,1 g | Wasser, vollentsalzt |
| 100,0 g | |

Vo der Anwendung werden 10 g Haarfärbelösung mit 10 g Wasserstoffperoxid-Lösung (6prozentige wäßrige Lösung) gemischt. Das erhaltene gebrauchsfertige Oxidationshaarfärbemittel wird auf durch Wind und Wetter geschädigte Haare aufgetragen. Nach einer Einwirkungszeit von 30 Minuten bei 40 °C werden die Haare mit Wasser gespült, schamponiert und getrocknet.

Das Haar hat einen gleichmäßig braun-orangen Farbton angenommen.

**Patentansprüche**

1. Haarfarbträgermasse enthaltend

- mindestens ein p-Aminophenolderivat, welches ausgewählt ist aus 4-Amino-2-methyl-phenol, 4-Amino-3-methyl-phenol, 4-Amino-2-hydroxymethyl-phenol, 4-Amino-2-methoxy-methyl-phenol und 4-Amino-2-aminomethyl-phenol,
- mindestens ein 5-Amino-2-methyl-phenol gemäß der allgemeinen Formel (I),

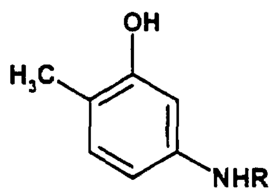

(I)

,

worin R Wasserstoff, eine geradkettige oder verzweigte Alkylgruppe mit 1 bis 4 Kohlenstoffatomen, eine geradkettige oder verzweigte Hydroxyalkylgruppe mit 2 bis 4 Kohlenstoffatomen oder eine geradkettige oder verzweigte Polyhydroxyalkylgruppe mit 3 bis 4 Kohlenstoffatomen bedeutet,

- mindestens ein m-Phenylendiaminderivat gemäß der allgemeinen Formel (II),

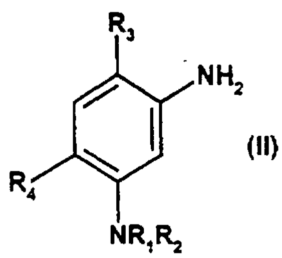

(II)

,

worin $R_1$ und $R_2$ unabhängig voneinander Wasserstoff, eine geradkettige oder verzweigte Alkylgruppe mit 1 bis 4 Kohlenstoffatomen, eine geradkettige oder verzweigte Hydroxyalkylgruppe mit 2 bis 4 Kohlenstoffatomen oder eine geradkettige oder verzweigte Polyhydroxyalkylgruppe mit 3 bis 4 Kohlenstoffatomen bedeuten,
$R_3$ Wasserstoff, eine geradkettige oder verzweigte Alkylgruppe mit 1 bis 6 Kohlenstoffatomen, eine geradkettige oder verzweigte Alkoxygruppe mit 1 bis 4 Kohlenstoffatomen, eine geradkettige oder verzweigte Hydroxyalkylgruppe mit 2 bis 4 Kohlenstoffatomen oder eine geradkettige oder verzweigte Polyhydroxyalkylgruppe mit 3 bis 4 Kohlenstoffatomen bedeutet, und

$R_4$ unabhängig von $R_1$, $R_2$ und $R_3$ Wasserstoff, eine geradkettige oder verzweigte Alkylgruppe mit 1 bis 6 Kohlenstoffatomen, eine geradkettige oder verzweigte Alkoxygruppe mit 1 bis 4 Kohlenstoffatomen, eine geradkettige oder verzweigte Hydroxyalkylgruppe mit 2 bis 4 Kohlenstoffatomen oder eine geradkettige oder verzweigte Polyhydroxyalkylgruppe mit 3 bis 4 Kohlenstoffatomen bedeutet,
und

- mindestens ein 2-Amino-6-chlor-4-nitrophenol gemäß der allgemeinen Formel (III),

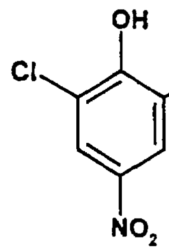

(III)

,

worin R' gleich Wasserstoff, einer geradkettigen oder verzweigten Alkylgruppe mit 1 bis 6 Kohlenstoffatomen, einer geradkettigen oder verzweigten Hydroxyalkylgruppe mit 2 bis 4 Kohlenstoffatomen oder einer geradkettigen oder verzweigten Polyhydroxyalkylgruppe mit 3 bis 4 Kohlenstoffatomen ist.

2. Haarfarbträgermasse nach Anspruch 1, **dadurch gekennzeichnet, daß** das 5-Amino-2-methyl-phenolderivat der Formel (1) ausgewählt ist aus 5-Amino-2-methyl-phenol, 5-Methylamino-2-methyl-phenol und 5-[(2'-Hydroxyethyl)-amino]-2-methyl-phenol.

3. Haarfarbträgermasse nach Anspruch 1 oder 2, **dadurch gekennzeichnet, daß** das m-Phenylendiaminderivat der Formel (II) ausgewählt ist aus 2-(2',4'-Diaminophenoxy)ethanol, 2,4-Diamino-1-(2',3'dihydroxypropoxy)benzol, 1,3-Bis(2',4'-diaminophenoxy)propan, 2-Amino-4-[(2'-hydroxyethyl)amino]anisol, 1,5-Bis(2'hydroxyethoxy)-2,4-diaminobenzol und m-Phenylendiamin.

4. Haarfarbträgermasse nach mindestens einem der Ansprüche 1 bis 3, **dadurch gekennzeichnet, daß** 2-Amino-6-chlor-4-nitrophenol der allgemeinen Formel (III) ausgewählt ist 2-Amino-6-chlor-4-nitrophenol, 2-Chlor-6-ethylamino-4-nitrophenol und 2-Chlor-6-((2'-hydroxyethyl)amino)-4-nitrophenol.

5. Haarfarbträgermasse nach mindestens einem der Ansprüche 1 bis 4, **dadurch gekennzeichnet, daß** sie zusätzlich weitere Oxidationsfarbvorstufen enthält, welche ausgewählt sind aus p-Phenylendiaminderivaten, Resorcinderivaten und 1,3-Benzodioxölderivaten.

6. Haarfarbträgermasse nach mindestens einem der Ansprüche 1 bis 5, **dadurch gekennzeichnet, daß** sie zusätzlich direktziehende Farbstoffe enthält.

7. Haarfarbträgermasse nach mindestens einem der Ansprüche 1 bis 6, **dadurch gekennzeichnet, daß** die Farbvorstufen in Form ihrer Additonssalze mit anorganischen oder organischen Säuren oder als Alkaliphenolate enthalten sind.

8. Haarfarbträgermasse nach mindestens einem der Ansprüche 1 bis 7, **dadurch gekennzeichnet, daß** die Farbstoffe in einem geeigneten kosmetischen Träger angewendet werden.

9. Haarfarbträgermasse nach mindestens einem der Ansprüche 1 bis 8, **dadurch gekennzeichnet, daß** die Farbvorstufen in einer Gesamtkonzentration von 0,1 und 10 Gewichtsprozent enthalten sind.

10. Haarfarbträgermasse nach mindestens einem der Ansprüche 1 der 9, **dadurch gekennzeichnet, daß** sie weitere übliche kosmetische Zusätze enthält.

11. Haarfärbemittel, **dadurch gekennzeichnet, daß** es unmittelbar vor der Anwendung durch Vermischen einer Haarfarbträgermasse nach mindestens einem der Ansprüche 1 bis 10 mit einem Oxidationsmittel hergestellt wird.

**12.** Haarfärbemittel nach Anspruch 11, **dadurch gekennzeichnet, daß** die Haarfarbträgermasse mit dem Oxidationsmittel im Verhältnis 5:1 bis 1:3 vermischt wird.

**13.** Haarfärbemittel nach Anspruch 11 oder 12, **dadurch gekennzeichnet, daß** es einen pH-Wert von 3 bis 11 aufweist.

**14.** Haarfärbeverfahren, **dadurch gekennzeichnet, daß** ein Haarfärbemittel nach mindestens einem der Ansprüche 11 bis 13 auf das Haar aufgetragen wird, bei einer Temperatur von 15 bis 50°C 10 bis 45 Minuten lang einwirken gelassen wird, das Haar anschließend mit Wasser gespült wird, gegebenenfalls schamponiert und sodann getrocknet wird.

## Claims

**1.** Hair dye carrier mass comprising

- at least one p-aminophenol derivative which is chosen from 4-amino-2-methylphenol, 4-amino-3-methylphenol, 4-amino-2-hydroxymethylphenol, 4-amino-2-methoxymethylphenol and 4-amino-2-aminomethylphenol,
- at least one 5-amino-2-methylphenol according to the general formula (I),

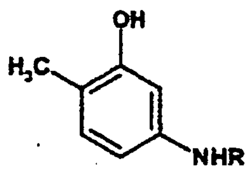

(I)

in which R is hydrogen, a straight-chain or branched alkyl group having 1 to 4 carbon atoms, a straight-chain or branched hydroxyalkyl group having 2 to 4 carbon atoms or a straight-chain or branched polyhydroxyalkyl group having 3 to 4 carbon atoms,
- at least one m-phenylenediamine derivative according to the general formula (II),

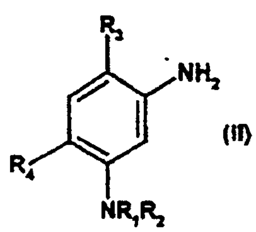

(II)

in which $R_1$ and $R_2$, independently of one another, are hydrogen, a straight-chain or branched alkyl group having 1 to 4 carbon atoms, a straight-chain or branched hydroxyalkyl group having 2 to 4 carbon atoms or a straight-chain or branched polyhydroxyalkyl group having 3 to 4 carbon atoms,
$R_3$ is hydrogen, a straight-chain or branched alkyl group having 1 to 6 carbon atoms, a straight-chain or branched alkoxy group having 1 to 4 carbon atoms, a straight-chain or branched hydroxyalkyl group having 2 to 4 carbon atoms or a straight-chain or branched polyhydroxyalkyl group having 3 to 4 carbon atoms, and
$R_4$, independently of $R_1$, $R_2$ and $R_3$, is hydrogen, a straight-chain or branched alkyl group having 1 to 6 carbon atoms, a straight-chain or branched alkoxy group having 1 to 4 carbon atoms, a straight-chain or branched hydroxyalkyl group having 2 to 4 carbon atoms or a straight-chain or branched polyhydroxyalkyl group having 3 to 4 carbon atoms,
and
- at least one 2-amino-6-chloro-4-nitrophenol according to the general formula (III),

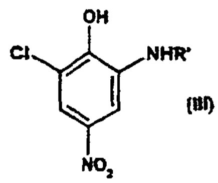

(III)

in which R' is hydrogen, a straight-chain or branched alkyl group having 1 to 6 carbon atoms, a straight-chain or branched hydroxyalkyl group having 2 to 4 carbon atoms or a straight-chain or branched polyhydroxyalkyl group having 3 to 4 carbon atoms.

**2.** Hair dye carrier mass according to Claim 1, **characterized in that** the 5-amino-2-methylphenol derivative of the formula (I) is chosen from 5-amino-2-methylphenol, 5-methylamino-2-methylphenol and 5-[(2'-hydroxyethyl)amino]-2-methylphenol.

**3.** Hair dye carrier mass according to Claim 1 or 2, **characterized in that** the m-phenylenediamine derivative of the formula (II) is chosen from 2-(2',4'-diaminophenoxy)ethanol, 2,4-diamino-1-(2',3'-dihydroxypropoxy)benzene, 1,3-bis(2',4'-diaminophenoxy)propane, 2-amino-4-[(2'-hydroxyethyl)amino]anisole, 1,5-bis(2'-hydroxyethoxy)-2,4-diaminobenzene and m-phenylenediamine.

**4.** Hair dye carrier mass according to at least one of Claims 1 to 3, **characterized in that** 2-amino-6-chloro-4-nitrophenol of the general formula (III) is chosen from 2-amino-6-chloro-4-nitrophenol, 2-chloro-6-ethylamino-4-nitrophenol and 2-chloro-6-((2'-hydroxyethyl)-amino)-4-nitrophenol.

**5.** Hair dye carrier mass according to at least one of Claims 1 to 4, **characterized in that** it additionally comprises further oxidation dye precursors which are chosen from p-phenylenediamine derivatives, resorcinol derivatives and 1,3-benzodioxole derivatives.

**6.** Hair dye carrier mass according to at least one of Claims 1 to 5, **characterized in that** it additionally comprises direct dyes.

**7.** Hair dye carrier mass according to at least one of Claims 1 to 6, **characterized in that** the dye precursors are present in the form of their addition salts with inorganic or organic acids or as alkali metal phenoxides.

**8.** Hair dye carrier mass according to at least one of Claims 1 to 7, **characterized in that** the dyes are used in a suitable cosmetic carrier.

**9.** Hair dye carrier mass according to at least one of Claims 1 to 8, **characterized in that** the dye precursors are present in a total concentration of from 0.1 to 10 per cent by weight.

**10.** Hair dye carrier mass according to at least one of Claims 1 to 9, **characterized in that** it comprises further customary cosmetic additives.

**11.** Hair dyeing agent **characterized in that** it is prepared directly prior to use by mixing a hair dye carrier mass according to at least one of Claims 1 to 10 with an oxidizing agent.

**12.** Hair dyeing agent according to Claim 11, **characterized in that** the hair dye carrier mass is mixed with the oxidizing agent in the ratio from 5:1 to 1:3.

**13.** Hair dyeing agent according to Claim 11 or 12, **characterized in that** it has a pH of from 3 to 11.

**14.** Hair dyeing method **characterized in that** a hair dyeing agent according to at least one of Claims 11 to 13 is applied to the hair, left to act at a temperature of from 15 to 50°C for from 10 to 45 minutes, and then the hair is rinsed with water, optionally shampooed and then dried.

## Revendications

1. Matière colorante pour cheveux, contenant

- au moins un dérivé de p-aminophénol qui est choisi parmi le 4-amino-2-méthylphénol, le 4-amino-3-méthylphénol, le 4-amino-2-hydroxyméthylphénol, le 4-amino-2-méthoxyméthylphénol et le 4-amino-2-aminométhylphénol,
- au moins un 5-amino-2-méthylphénol correspondant à la formule générale (I),

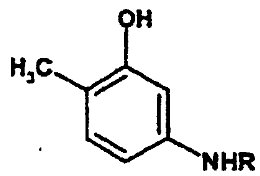

(I)

dans laquelle R représente un atome d'hydrogène, un groupe alkyle à chaîne droite ou ramifiée ayant de 1 à 4 atomes de carbone, un groupe hydroxyalkyle à chaîne droite ou ramifiée ayant de 2 à 4 atomes de carbone ou un groupe polyhydroxyalkyle à chaîne droite ou ramifiée ayant 3 ou 4 atomes de carbone,
- au moins un dérivé de m-phénylènediamine correspondant à la formule générale (II),

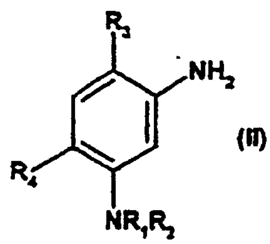

(II)

dans laquelle $R_1$ et $R_2$ représentent, indépendamment l'un de l'autre, un atome d'hydrogène, un groupe alkyle à chaîne droite ou ramifiée ayant de 1 à 4 atomes de carbone, un groupe hydroxyalkyle à chaîne droite ou ramifiée ayant de 2 à 4 atomes de carbone ou un groupe polyhydroxyalkyle à chaîne droite ou ramifiée ayant 3 ou 4 atomes de carbone,
$R_3$ représente un atome d'hydrogène, un groupe alkyle à chaîne droite ou ramifiée ayant de 1 à 6 atomes de carbone, un groupe alcoxy à chaîne droite ou ramifiée ayant de 1 à 4 atomes de carbone, un groupe hydroxyalkyle à chaîne droite ou ramifiée ayant de 2 à 4 atomes de carbone ou un groupe polyhydroxyalkyle à chaîne droite ou ramifiée ayant 3 ou 4 atomes de carbone, et
$R_4$ représente, indépendamment de $R_1$, $R_2$ et $R_3$, un atome d'hydrogène, un groupe alkyle à chaîne droite ou ramifiée ayant de 1 à 6 atomes de carbone, un groupe alcoxy à chaîne droite ou ramifiée ayant de 1 à 4 atomes de carbone, un groupe hydroxyalkyle à chaîne droite ou ramifiée ayant de 2 à 4 atomes de carbone ou un groupe polyhydroxyalkyle à chaîne droite ou ramifiée ayant 3 ou 4 atomes de carbone,
et
- au moins un 2-amino-6-chloro-4-nitrophénol correspondant à la formule générale (III),

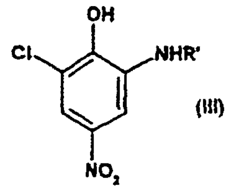

(III)

dans laquelle R' représente un atome d'hydrogène, un groupe alkyle à chaîne droite ou ramifiée ayant de 1

à 6 atomes de carbone, un groupe hydroxyalkyle à chaîne droite ou ramifiée ayant de 2 à 4 atomes de carbone ou un groupe polyhydroxyalkyle à chaîne droite ou ramifiée ayant 3 ou 4 atomes de carbone.

**2.** Matière colorante pour cheveux selon la revendication 1, **caractérisée en ce que** le dérivé de 5-amino-2-méthylphénol de formule (I) est choisi parmi le 5-amino-2-méthylphénol, le 5-méthylamino-2-méthylphénol et le 5-[(2'-hydroxyéthyl)amino]-2-méthylphénol.

**3.** Matière colorante pour cheveux selon la revendication 1 ou 2, **caractérisée en ce que** le dérivé de m-phénylènediamine de formule (II) est choisi parmi le 2-(2',4'-diaminophénoxy)éthanol, le 2,4-diamino-1-(2',3'-dihydroxypropoxy)benzène, le 1,3-bis(2',4'-diaminophénoxy)propane, le 2-amino-4-[(2'-hydroxyéthyl)amino]anisole, le 1,5-bis(2'-hydroxyéthoxy)-2,4-diaminobenzène et la m-phénylènediamine.

**4.** Matière colorante pour cheveux selon au moins l'une des revendications 1 à 3, **caractérisée en ce que** le 2-amino-6-chloro-4-nitrophénol de formule générale (III) est choisi parmi le 2-amino-6-chloro-4-nitrophénol, le 2-chloro-6-éthylamino-4-nitrophénol et le 2-chloro-6-((2'-hydroxyéthyl)amino)-4-nitrophénol.

**5.** Matière colorante pour cheveux selon au moins l'une des revendications 1 à 4, **caractérisée en ce qu'**elle contient en outre d'autres précurseurs de colorants d'oxydation qui sont choisis parmi des dérivés de p-phénylènediamine, des dérivés de résorcinol et des dérivés de 1,3-benzodioxole.

**6.** Matière colorante pour cheveux selon au moins l'une des revendications 1 à 5, **caractérisée en ce qu'**elle contient en outre des colorants directs.

**7.** Matière colorante pour cheveux selon au moins l'une des revendications 1 à 6 **caractérisée en ce que** les précurseurs de colorants sont contenus sous forme de leurs sels d'addition avec des acides organiques ou minéraux ou sous forme de phénolates alcalins.

**8.** Matière colorante pour cheveux selon au moins l'une des revendications 1 à 7, **caractérisée en ce que** les colorants sont utilisés dans un véhicule cosmétique approprié.

**9.** Matière colorante pour cheveux selon au moins l'une des revendications 1 à 8, **caractérisée en ce que** les précurseurs de colorants sont utilisés à une concentration totale de 0,1 à 10 % en poids.

**10.** Matière colorante pour cheveux selon au moins l'une des revendications 1 à 9, **caractérisée en ce qu'**elle contient d'autres additifs cosmétiques usuels.

**11.** Produit de teinture pour cheveux, **caractérisé en ce qu'**il est préparé immédiatement avant l'emploi, par mélange d'une matière colorante pour cheveux selon au moins l'une des revendications 1 à 10, avec un oxydant.

**12.** Produit de teinture pour cheveux selon la revendication 11, **caractérisé en ce que** la matière colorante pour cheveux est mélangée avec l'oxydant en un rapport de 5:1 à 1:3.

**13.** Produit de teinture pour cheveux selon la revendication 11 ou 12, **caractérisé en ce qu'**il présente un pH de 3 à 11.

**14.** Procédé de teinture des cheveux, **caractérisé en ce qu'**on applique sur les cheveux un produit de teinture pour cheveux selon au moins l'une des revendications 11 à 13, on laisse agir pendant 10 à 45 minutes à une température de 15 à 50°C, puis on rince les cheveux à l'eau, éventuellement on les shampooine et ensuite on les sèche.